# EUROPEAN PATENT APPLICATION

(11) **EP 4 407 040 A1**
(43) Date of publication of application: **31.07.2024**
(21) Application number: 22872814.3
(22) Date of filing: 15.09.2022
(51) Int. Cl.: C12Q 1/06, C12N 1/00, C12N 5/077, C12N 15/12

(54) **SCREENING METHOD USING BROWN ADIPOCYTES**

(30) Priority: 21.09.2021 JP 2021153080
(71) Applicant: Kataoka Corporation, Kyoto-shi, Kyoto 601-8203 (JP); Kyoto Prefectural Public University Corporation, Kyoto-shi, Kyoto 602-8566 (JP)
(72) Inventor: DAI, Ping, Kyoto-shi Kyoto 602-8566 (JP); TAKEDA, Yukimasa, Kyoto-shi Kyoto 602-8566 (JP); HARADA, Yoshinori, Kyoto-shi Kyoto 602-8566 (JP); MATSUMOTO, Junichi, Kyoto-shi Kyoto 601-8203 (JP)
(74) Representative: Vossius & Partner Patentanwälte Rechtsanwälte mbB
(86) International application number: PCT/JP2022/034540
(87) International publication number: WO 2023/048060

(57) **Abstract**

The present invention is to provide a new screening method to search for compounds that have browning-promoting or browning-inhibiting effects on brown adipocytes and that target cell membrane receptors (e.g., TRPV1, TRPV4, etc.) based on evaluation of browning tendency of low-molecular-induced brown adipocytes directly converted or induced from somatic cells by low-molecular compounds, without gene transfer.

The present invention, for example, can include a screening method of searching for a compound targeting a cell membrane receptor and having a browning-promoting or browning-inhibiting effect on brown adipocytes, wherein the method comprises a step of measuring the browning index of low-molecular-weight compound-induced brown adipocytes with or without a candidate compound applied to the brown adipocytes.

The present invention allows candidate compounds that can act on brown adipocytes via TRPV1 or TRPV4 to be found.

## Description

### Technical Field

### (Cross-reference to related applications)

The present application claims the benefit of Japanese Application No. 2021-153080, filed September 21, 2021 with the Japanese Patent Office. The Japanese application is hereby incorporated by reference for all purposes as if the entirety of its application documents (specification, claims, drawings, and abstract) were expressly set forth herein.

The present invention belongs to the technical field of low-molecular-weight compound-induced brown adipocytes (ciBAs: chemical compound-induced Brown Adipocytes). In the technical field, the present invention relates to a screening method of searching for compounds (including bioactive substances) that have browning-promoting or browning-inhibiting effects on brown adipocytes. The present invention further relates to a method for producing ciBA and an induction medium composition that can be used for the method for producing the ciBA.

### Background of the Invention

In recent years, methods, i.e., Direct Reprogramming, Direct Conversion, or Direct Conversion, for converting somatic cells, such as fibroblasts, directly into other cell types without gene transfer have been actively studied. For example, Non-Patent Document 1 discloses a method of producing low molecular weight compound-induced brown adipocytes (ciBA) by direct induction (direct conversion) by culturing somatic cells in the presence of low-molecular-weight compounds such as ALK5 inhibitors, ALK2/3 inhibitors, ALK2/3/6 inhibitors, cAMP inducers, and PPARγ agonists. In addition, Patent Document 1 and Non-Patent Document 2 disclose a method of producing ciBA by direct induction (direct conversion) from somatic cells in a serum-free differentiation induction medium in the presence of a selective PPARγ agonist and a cAMP inducer, or in addition to them, in the presence of BMP7. Both of the documents and Patent Document 2 disclose compositions for a medium that can be used in the ciBA production method.

On the other hand, it is known that various cell membrane receptors are expressed on cell surfaces in the body. Such receptors include, for example, TRPV1 and TRPV4. TRPV1 or TRPV4 belongs to the TRPV (Transient Receptor Potential Vanilloid) subfamily of the temperature-sensitive TRP channel superfamily. TRPV1 is expressed in the sensory nerve endings of the gastrointestinal tract and is one of the major receptors for exerting anti-obesity effects through activation of the sympathetic nervous system by transmitting stimuli from food components such as capsaicin, a component of Capsicum annuum, to the central nervous system.

Anti-obesity effects of continuous capsaicin intake have been observed, for example, reduction of body fat, reduction of blood glucose levels, and enhancement of energy expenditure. However, there are few reports on the direct effects of capsaicin on brown adipocytes (e.g., Non-Patent Document 3, 4). In particular, the direct effect of capsaicin on human brown adipocytes is largely unknown.

TRPV4 is expressed in many tissues, including sensory nerves, kidney, skin, blood vessels, lung, and fat, and is known to be activated by physical stimuli and arachidonic acid metabolites. In particular, TRPV4 expressed in adipocytes has been reported to inhibit browning (Non-Patent Document 5).

### Prior Art

### Patent Document

Patent Document 1: WO 2021/106697
Patent Document 2: WO 2021/106698

### Non-Patent Document

Non-Patent Document 1: TakedaY., HaradaY., Yoshikawa T., and Dai P., Sci. Rep.,7, 4304 (2017)
Non-Patent Document 2: Takeda Y. and Dai P., Sci. Rep., 10, 3775 (2020)
Non-Patent Document 3: Kida R., et al., Cell Biochem. Func. 34, 34-41 (2016)
Non-Patent Document 4: Kida R., et al., Sci. Rep. 8, 845 (2018)
Non-Patent Document 5: Ye L., Spiegelman B.M., et al., Cell, 151, 96-110 (2012)

### Summary of the Invention

### Problem to be Solved by the Invention

The present invention is primarily directed to provide a new screening method to search for compounds that have browning-promoting or browning-inhibiting effects on brown adipocytes and that target cell membrane receptors (e.g., TRPV1, TRPV4, etc.) based on the evaluation of browning tendency of low-molecular-weight compound-induced brown adipocytes directly converted or induced from somatic cells by low-molecular-weight compounds, without gene transfer.

### Means for Solving the Problems

As a result of diligent study, the present inventors have found that it is possible to search for compounds that target cell membrane receptors and that have a browning-promoting or browning-inhibiting effect on brown adipocytes, by evaluating the tendency to turn brown of low-molecular-weight compound-induced brown adipocytes and have completed the present invention.

The present invention, for example, can include the following embodiments.
[1] A screening method of searching for a compound targeting a cell membrane receptor and having a browning-promoting or browning-inhibiting effect on brown adipocytes, wherein the method comprises a step of measuring the browning index of low-molecular-weight compound-induced brown adipocytes with and without a candidate compound applied to the brown adipocytes.
[2] The screening method according to the [1] mentioned above, wherein the compound having a browning-promoting or browning-inhibiting effect is a candidate compound that can act on brown adipocytes via TRPV1 or TRPV4.
[3] The screening method according to the [1] or [2] mentioned above, wherein the low-molecular-weight compound-induced brown adipocytes are induced to differentiate directly from a somatic cell by comprising a step of culturing the somatic cell in a serum-free differentiation induction medium in which a selective PPARγ agonist and a cAMP inducer are present.
[4] The screening method according to the [3] mentioned above, wherein the step comprises a step of culturing a somatic cell in the presence of BMP7.
[5] The screening method according to the [3] or [4] mentioned above, wherein the selective PPARγ agonist comprises rosiglitazone or the cAMP inducer comprises forskolin.
[6] The screening method according to any one of the [3] to [5] mentioned above, wherein the somatic cell is a fibroblast.
[7] The screening method according to any one of the [1] to [6] mentioned above, wherein the browning index is one or more selected from the group consisting of Ucp1 gene expression, Fabp4 gene expression, Cidea gene expression, induction efficiency of low-molecular-weight compound-induced brown adipocytes, amount of mitochondrial biosynthesis, oxygen consumption, extracellular acidification rate, amount of glycerol secretion, and amount of triglyceride accumulation.
[8] A method of producing low-molecular-weight compound-induced brown adipocytes by inducing differentiation directly from a somatic cell using a compound targeting a cell membrane receptor and having a browning-promoting effect on brown adipocytes, wherein the method comprises a step of culturing a somatic cell in a serum-free differentiation induction medium in which the compound, a selective PPARγ agonist, and a cAMP inducer are present.
[9] The method of producing brown adipocytes according to the [8] mentioned above, wherein the compound having a browning-promoting effect comprises a TRPV1 agonist or a TRPV4 antagonist.
[10] The method of producing brown adipocytes according to the [8] or [9] mentioned above, wherein the step comprises a step of culturing a somatic cell in the presence of BMP7.
[11] The method of producing brown adipocytes according to the [9] or [10] mentioned above, wherein the selective PPARγ agonist comprises rosiglitazone, the cAMP inducer comprises forskolin, or the TRPV1 agonist comprises capsaicin.
[12] The method of producing brown adipocytes according to any one of the [8] to [11] mentioned above, wherein the somatic cell is fibroblast.
[13] An inducing differentiation composition for producing low-molecular-weight compound-induced brown adipocytes by inducing differentiation directly from a somatic cell using a compound targeting a cell membrane receptor and having a browning-promoting effect on brown adipocytes, wherein the composition comprises the compound, a selective PPARγ agonist, and a cAMP inducer, and is serum-free.
[14] The inducing differentiation composition according to the [13] mentioned above, wherein the compound having a browning-promoting effect comprises a TRPV1 agonist or a TRPV4 antagonist.
[15] The inducing differentiation composition according to the [13] or [14] mentioned above, further comprising BMP7.
[16] The inducing differentiation composition according to the [14] or [15] mentioned above, wherein the selective PPARγ agonist comprises rosiglitazone, the cAMP inducer comprises forskolin, or the TRPV1 agonist comprises capsaicin.
[17] The inducing differentiation composition according to any one of the [13] to [16] mentioned above, wherein the somatic cell is fibroblast.

### Effect of the Invention

The present invention allows compounds that target cell membrane receptors such as TRPV1 and TRPV4 and that promote or inhibit browning to brown adipocytes to be found. In addition, direct conversion or direct differentiation induction from somatic cells to brown adipocytes can be performed more effectively in serum-free conditions without gene transfer.

### Brief Description of the Drawings

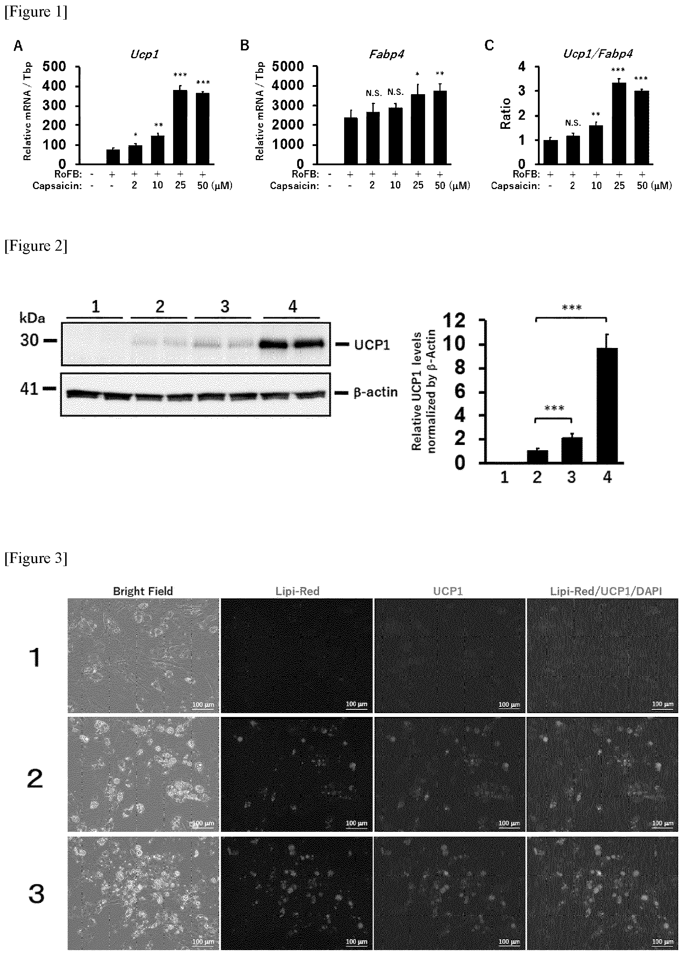

[Figure 1] Figure A shows the expression level of the Ucp1 gene, Figure B shows the expression level of the Fabp4 gene, and Figure C shows its Ucp1/Fabp4 ratio. In the figures, "RoFB" indicates the combination of rosiglitazone, forskolin, and BMP7, "+" indicates that the culture was grown in the presence of RoFB, and "-" indicates that the culture was grown in the absence of RoFB or capsaicin. The vertical axis indicates the relative value of the mRNA amount relative to that of the internal standard gene (Tbp).
[Figure 2] The left figure is an electrophoresis photograph of the quantified UCP1 protein. The right figure shows the quantified band intensity of the electrophoresis. In the figure, Symbol 1 shows the results of incubation in the absence of rosiglitazone, forskolin, BMP7, and capsaicin, Symbol 2 shows the results of incubation in the presence of rosiglitazone, forskolin, and BMP7, and Symbol 3 shows the results of incubation in the presence of rosiglitazone, forskolin, BMP7, and capsaicin (5 µmol/L), and Symbol 4 shows the results when cultured in the presence of rosiglitazone, forskolin, BMP7, and capsaicin (25 µmol/L). In the right figure, the vertical axis indicates the amount of UCP1 protein relative to the amount of internal standard protein β-actin.
[Figure 3] Photographs of cultured cells and immunostaining of fat droplets (red), UCP1 protein (green), and cell nuclei (blue) of the cells are represented. Since Figure 3 is in black and white, the red, green and blue colors are not shown, but each color is shown in the original photograph. Symbol 1 shows the results of incubation in the absence of rosiglitazone, forskolin, BMP7, and capsaicin; Symbol 2 shows the results of incubation in the presence of rosiglitazone, forskolin, and BMP7; and Symbol 3 shows the results of incubation in the presence of rosiglitazone, forskolin, BMP7, and capsaicin (25 µmol/L).

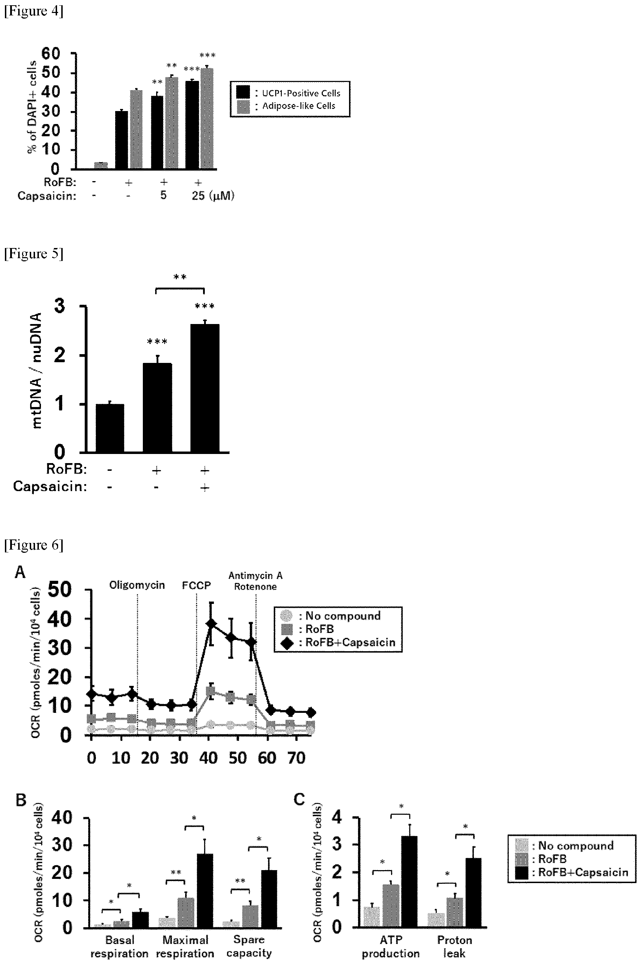

[Figure 4] The rate of adipose-like cells and UCP1-positive cells is represented. In the figure, "RoFB" indicates the combination of rosiglitazone, forskolin, and BMP7, "+" indicates a culture in the presence of RoFB, and "-" indicates a culture in the absence of RoFB or capsaicin. The vertical axis indicates the percentage (%) of these cells relative to cells positive for cell nuclear staining (DAPI).
[Figure 5] The relative amounts of mitochondrial DNA are represented. In the figure, "RoFB" indicates the combination of rosiglitazone, forskolin, and BMP7, "+" indicates a culture in the presence of RoFB or capsaicin (25 µmol/L), and "-" indicates a culture in the absence of RoFB or capsaicin. The vertical axis indicates the relative amount of mitochondrial DNA (mtDNA) to the amount of cellular nuclear DNA (nuDNA).
[Fig. 6] Figure A represents the rate of oxygen consumption. The light gray circles represent the results in control cells (without addition of RoFB and capsaicin), gray squares represent the results when cultured in the presence of RoFB, and black diamonds represent the results when cultured in the presence of RoFB and capsaicin. In the figure, the dotted line indicates the addition of oligomycin, FCCP or antimycin A/rotenone. The horizontal axis indicates time (minutes); Figure B and C represent the oxygen consumption rate corresponding to each parameter on the horizontal axis. In the results for each parameter, each left column represents the results of incubation in the absence of both RoFB and capsaicin, each middle column represents the results of incubation in the presence of RoFB, and each right column represents the results of incubation in the presence of RoFB and capsaicin. The vertical axis represents the oxygen consumption rate OCR (picomoles/min/10⁴ cells).

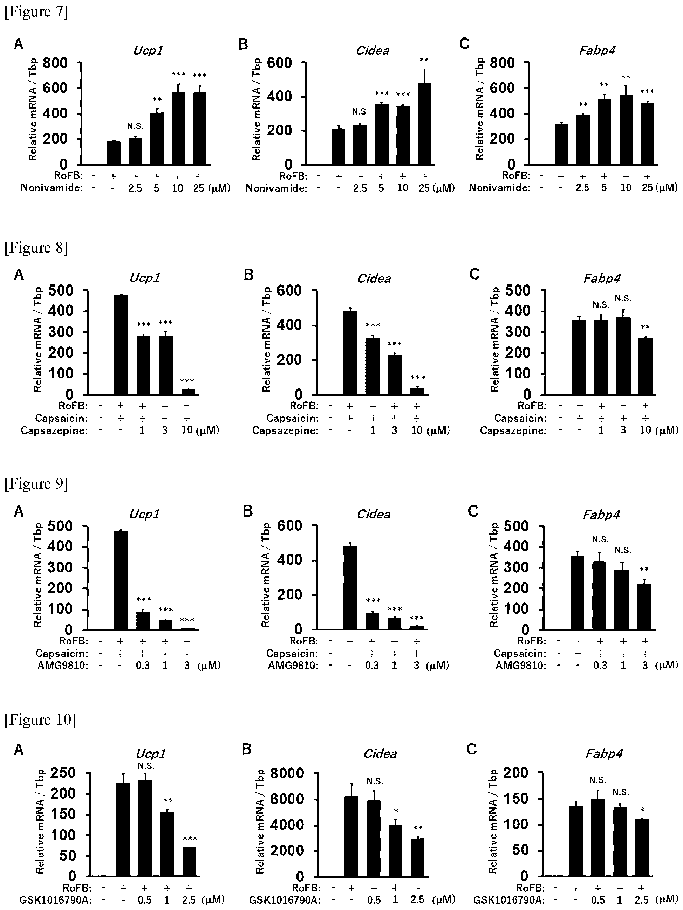

[Figure 7] Figure A shows the expression level of the Ucp1 gene, Figure B shows the expression level of the Cidea gene, and Figure C shows the expression level of Fabp4. In the figures, "RoFB" indicates the combination of rosiglitazone, forskolin, and BMP7, "+" indicates a culture in the presence of RoFB, and "-" indicates a culture in the absence of RoFB or nonivamide. The vertical axis indicates the relative value of the mRNA amount relative to the mRNA amount of the internal standard gene (Tbp).
[Fig. 8] Figure A represents the expression level of Ucp1 gene, Figure B represents the expression level of Cidea gene, and Figure C represents the expression level of Fabp4. In the figures, "RoFB" indicates the combination of rosiglitazone, forskolin, and BMP7, "+" indicates a culture in the presence of RoFB or capsaicin (25 µmol/L), and "-" indicates a culture in the absence of RoFB, capsaicin, or capsazepine. The vertical axis indicates the relative value of the mRNA amount relative to the mRNA amount of the internal standard gene (Tbp).
[Fig. 9] Figure A represents the expression level of Ucp1 gene, Figure B represents the expression level of Cidea gene, and Figure C represents the expression level of Fabp4. In the figures, "RoFB" indicates the combination of rosiglitazone, forskolin, and BMP7, "+" indicates a culture in the presence of RoFB or capsaicin (25 µmol/L), "-" indicates a culture in the absence of RoFB, capsaicin, or AMG9810. The vertical axis indicates the mRNA levels relative to the mRNA levels of the internal standard gene (Tbp).
[Fig. 10] Figure A shows the expression level of Ucp1 gene, Figure B shows the expression level of Cidea gene, and Figure C shows the expression level of Fabp4. In the figures, "RoFB" indicates the combination of rosiglitazone, forskolin, and BMP7, "+" indicates a culture in the presence of RoFB, and "-" indicates a culture in the absence of RoFB or GSK1016790A. The vertical axis indicates the relative value of the mRNA amount relative to the mRNA amount of the internal standard gene (Tbp).

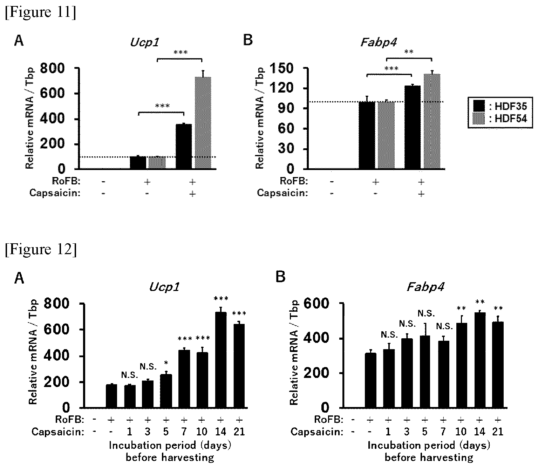

[Fig. 11] Figures A and B show the expression levels of the Ucp1 and Fabp4 genes, respectively. In the figures, "RoFB" indicates the combination of rosiglitazone, forskolin, and BMP7, "+" indicates a culture in the presence of RoFB or capsaicin (25 µmol/L), and "-" indicates a culture in the absence of RoFB or capsaicin. In the results of dermal fibroblasts derived from different human specimens, each left column represents the results ofHDF35 and each right column represents the results ofHDF54. The vertical axis indicates the relative value of the mRNA amount relative to the mRNA amount of the internal standard gene (Tbp).
[Fig. 12] Figures A and B represent the expression levels of the Ucp1 and Fabp4 genes, respectively. The figures represent the results of incubation with 25 µM capsaicin for 1, 3, 5, 7, 10, 14, and 21 days, respectively, before cells were collected on day 21 after induction. In the figures, "RoFB" indicates the combination of rosiglitazone, forskolin, and BMP7, "+" indicates a culture in the presence of RoFB, and "-" indicates a culture in the absence of RoFB or capsaicin. The vertical axis indicates the relative value of the mRNA amount relative to the mRNA amount of the internal standard gene (Tbp).

### Mode for Carrying Out the Present Invention

Hereinafter, the present invention will be described in detail.

### 1 Screening method for the present invention

The screening method of the present invention (hereinafter referred to as "the present screening method") is characterized in that it is a screening method of searching for a compound that targets a cell membrane receptor and that has a browning-promoting or browning-inhibiting effect on brown adipocytes , wherein the method comprises a step of measuring the browning index of low-molecular-weight compound-induced brown adipocytes (ciBA) with and without a candidate compound applied to the brown adipocytes.

The term "browning" refers not only to the transformation of white adipocytes or adipose precursor cells into brown adipocytes (beige adipocytes), but also to the maturation of the brown adipocytes (beige cells) themselves. Such maturation can, for example, enhance glucose metabolism, conversion of fatty acids to thermal energy, mitochondrial biosynthesis, and gene expression related to these processes in brown adipocytes. The present screening method allows compounds that have an effect of promoting or inhibiting such maturation to be preferably searched for. The tendency of browning can be evaluated by the browning index described below.

In addition, "compounds having a browning-promoting or browning-inhibiting effect (on brown adipocytes)" means all compounds (substances) that have the above effects of promoting or inhibiting browning, and such compounds include low-, medium-, and high-molecular-weight compounds, as well as coordination compounds, complexes, and supramolecules. It also includes so-called bioactive substances such as proteins (cytokines, etc.), lipids, and active ingredients in extracts.

The cell membrane receptors targeted by the above compounds, which are the searching subjects of the screening method, are not particularly limited, as long as they are expressed on the surface of brown adipocytes, white adipocytes, or adipose progenitor cells and can be involved in promoting or inhibiting browning. Such receptors include, for example, TRPV1, TRPV2, TRPV3, TRPV4, TRPA1, TRPM4, TRPM8, TRPC5, β-adrenergic receptor, TGR5 bile acid receptor, and adenosine receptor. The present screening method allows compounds that target at least one of these and other cell membrane receptors and that have a browning-promoting or browning-inhibiting effect on brown adipocytes to be found.

It is known that ciBA can be induced directly from somatic cells, for example, by culturing somatic cells under a serum-free differentiation induction medium in the presence of selective PPARγ agonists and cAMP inducers, or preferably in addition to these, and also BMP7 (e.g., Patent-Documents 1 and 2; Non-Patent Document 2).

Therefore, since ciBA is gradually induced from somatic cells by the above culture, the candidate compound can be applied to ciBA by including the candidate compound in the culture from the start of the culture or at an appropriate time after the start of the culture. The browning index of the ciBA obtained in the case where the candidate compound is included in the culture and acts on the ciBA, and in the case where the candidate compound is not included and does not act on the ciBA, is measured, and evaluation of the browning tendency by comparison enables a judgement, for example, whether or not the candidate compound is a candidate compound that can act on brown adipocytes via TRPV1 or TRPV4.

Specifically, as is clear from the examples described below, the TRPV1 agonist capsaicin promotes adipocyte browning and the TRPV4 agonist GSK1016790A inhibits adipocyte browning, so TRPV1 and TRPV4 are both receptors that can be involved in browning. Receptors such as TRPV2, TRPV3, TRPA1, TRPM4, TRPM8, TRPC5, β-adrenergic receptor, TGR5 bile acid receptor, and adenosine receptor can also be involved in browning as well. If browning is enhanced or inhibited when cultured with a candidate compound compared to when cultured without the candidate compound, it can be determined that the candidate compound targets at least one of the cell membrane receptors, including the receptors listed above, and has a browning-promoting or browning-inhibiting effect on brown adipocytes. Alternatively, for example, if browning is enhanced when cultured with a candidate compound compared to when cultured without the candidate compound, it can be determined that the candidate compound may be a TRPV 1 agonist or TRPV4 antagonist. Conversely, if browning is inhibited more when cultured with a candidate compound than when cultured without the candidate compound, it can be determined that the candidate compound may be a TRPV1 antagonist or a TRPV4 agonist.

The amount of a candidate compound added in the system when it is included can be set as desired, but it is preferable to evaluate the browning tendency in a concentration-dependent manner.

The browning index for the present invention includes, for example, Ucp1 gene expression, Fabp4 gene expression, Cidea gene expression, efficiency of induction of low molecular weight compound-induced brown adipocytes, mitochondrial biosynthesis, oxygen consumption, extracellular acidification rate, glycerol secretion, and triglyceride accumulation. One or more of these can be used in combination to evaluate the browning tendency of the obtained ciBA, but it is preferable to obtain two or more indicator data to evaluate the browning tendency. For example, the expression levels of the Ucp1 gene, which is a marker specific to brown adipocytes, and the Fabp4 gene, which is expressed in adipocytes in general, can be quantified by real-time PCR, for example, and the ratio of the two can be used as an indicator of the browning tendency.

Candidate compounds in the present screening method include, for example, known medicines in so-called lifestyle-related disease medicines, metabolic disease medicines, and other library medicines, as well as a wide range of food nutrients such as vitamins, sugars, amino acids, fatty acids, lipids and minerals, components of functional foods, herbal medicine, herbs, other low molecular weight compounds in foods, and proteins including cytokines.

### 2 Direct differentiation induction from somatic cells to ciBAs

The method of producing a ciBA according to the present invention (hereinafter referred to as the "production method of the present invention") is characterized in that a ciBA is directly induced from a somatic cell by culturing the somatic cell under a serum-free differentiation inducing medium in which a compound having a browning-promoting effect (e.g., TRPV1 agonists, TRPV4 antagonists.) ), a selective PPARγ agonist, or a cAMP inducer is present, or preferably in addition to these, BMP7 is present. The production method of the present invention allows ciBAs to be induced without exogenous gene transfer. That is, the production method of the present invention may not include the step of gene transfer.

Here, "serum-free" means substantially free of unadjusted or unpurified serum. Even if purified blood-derived components or animal tissue-derived components are mixed in differentiation induction mediums, it can be called serum-free differentiation induction mediums as long as it contains substantially no unadjusted or unpurified serum.

A "gene" means a substance (molecule or complex thereof) that has a chemical structure containing multiple nucleobases and can be involved in carrying, transcription, or translation of genetic information or inhibition thereof, and includes both DNA and RNA. In addition, "gene transfer" includes the introduction of DNA by viral vectors, etc., as well as the introduction of RNA (siRNA, miRNA, piRNA, etc.) for the purpose of RNA silencing (RNA interference, etc.) and all other events in which a foreign gene is introduced into a cell. The production method of the present invention allows the possibility (risk) of unexpected genome mutation and insertion, as well as tumorigenesis, or the like to extremely reduce, because a ciBA can be induced without gene transfer.

Selective PPARγ agonists and cAMP inducers may be used one or more in combination in each.

### 2.1 Somatic Cells

Cells of an organism can be classified into somatic and germ cells. Any somatic cell can be used as a starting material in the present invention. The somatic cell is not particularly limited, and may be either a primary cell taken from a living body or a cell from an established cell line. Somatic cells at various stages of differentiation, e.g., terminally differentiated somatic cells (e.g., fibroblasts, umbilical vein endothelial cells (HUVEC), hepatic cells (Hepatocytes), bile duct cells (Biliary cells), pancreatic alpha cells (Pancreatic α cells), pancreatic acinar cells (Acinar cells), pancreatic ductal glandular cells (Ductal cells), small intestinal crypt cells (Intestinal crypt cells), etc.), somatic cells on the way to terminal differentiation (e.g., mesenchymal stem cells, neural stem cells, endodermal progenitor cells, etc.), or such somatic cells like iPS cells that have been initialized to acquire pluripotency and somatic cells that have differentiated or are in the process of differentiation from iPS cells, can be used in the present invention. The somatic cells that can be used in the present invention include any somatic cells, for example, cells of the hematopoietic system (various lymphocytes, macrophages, dendritic cells, bone marrow cells, etc.), cells derived from organs (hepatocytes, splenocytes, pancreatic cells, kidney cells, lung cells, etc.), cells of the muscle tissue system (skeletal muscle cells, smooth muscle cells, myoblasts, cardiomyocytes, etc.), fibroblasts, neurons, osteoblasts, chondrocytes, endothelial cells, interstitial cells, adipocytes (white adipocytes, etc.), embryonic stem cells (ES cells), etc. The present invention may also be the precursor cells of these cells and cancer cells. Fibroblasts are preferred.

Fibroblasts that can be used in the present invention are not particularly limited, and examples of the fibroblasts can include skin fibroblasts (Dermal Fibroblasts), aortic outer membrane fibroblasts (Adventitial Fibroblasts), cardiac fibroblasts (Cardiac Fibroblasts), pulmonary fibroblasts (Pulmonary Fibroblasts), uterine fibroblasts (Uterine Fibroblasts), chorionic mesenchymal fibroblasts (Villous Mesenchymal Fibroblasts), all of which are primary cell components constituting connective tissues in various tissues and organs, and are cells producing collagen fibers.

Examples of the source of the above-mentioned somatic cells include, but are not limited to, humans, mammals other than humans, and animals other than mammals (birds, reptiles, amphibians, fishes, and the like). As a source of somatic cells, humans and mammals other than humans are preferred, and humans are particularly preferred. When brown adipocytes are produced for the purpose of administration to humans, preferably, somatic cells harvested from a donor having a matched or similar type of histocompatibility antigen to the recipient can be used. Somatic cells harvested from the recipient itself may be used.

### 2.2 Selective PPARγ agonists

PPARγ (Peroxisome proliferator-activated Receptor γ) is one of the proteins belonging to the nuclear receptor superfamily and is a transcription factor that controls gene expression by forming heterodimers with retinoid X receptors (RXRs) and binding to the promoter regions of target genes. There are three subtypes of PPARs: α, δ (β), and γ, and the present invention uses an agonist that selectively acts on γ.

The term "in the presence of a selective PPARγ agonist" refers to a culture condition under which an agonist action can be selectively exerted on PPARγ, and the means is not particularly limited, and a compound which selectively exerts an agonist action on PPARγ can be usually utilized.

Although not particularly limited in the present invention, examples of the selective PPARγ agonist include the following compounds or salts thereof. Preference is given to selective PPARγ agonists of the thiazolidine system, in particular rosiglitazone, pioglitazone, and troglitazone.

### Rosiglitazone (CAS No.: 122320-73-4)

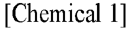

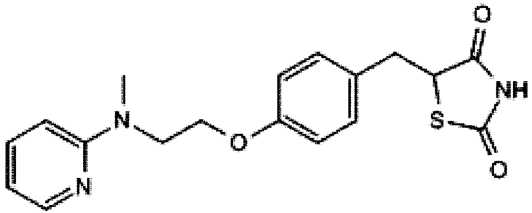

Rosiglitazone maleate (CAS No.: 155141-29-0)
Ciglitazone (CAS No.: 74772-77-3)
Edaglitazone (CAS No.: 213411-83-7)
GW1929 hydrochloride (CAS No.: 1217466-21-1)
nTZDpa (CAS No.: 118414-59-8)
Pioglitazone hydrochloride (CAS No.: 112529-15-4)
S26948 (CAS No.: 353280-43-0)
Troglitazone (CAS No.: 97322-87-7)
Balaglitazone (CAS No.: 199113-98-9)
Rivoglitazone (CAS No.: 185428-18-6)

The concentration of the selective PPARγ agonist varies depending on the kind of the agonist, the somatic cell used, and the like, but is not particularly limited and may be appropriately determined, and may be used in a range of, for example, 0.1 µmol/L to 20 µmol/L, preferably 0.5 µmol/L to 5 µmol/L.

### 2.3 cAMP Inducers

Cyclic adenosine monophosphate (cAMP) is a second messenger that is involved in a variety of intracellular signaling events. The cAMP is produced intracellularly by cyclization of adenosine triphosphate (ATP) by adenylyl cyclase (adenylate cyclase).

The term "in the presence of a cAMP inducer" refers to a culture condition capable of inducing cAMP, and the means thereof is not particularly limited, and for example, any means capable of increasing the intracellular cAMP level can be used. Adenylate cyclase activators such as substances that can act directly on and induce adenylate cyclase, an enzyme involved in the generation of cAMP, substances that can promote the expression of adenylate cyclase, neurotransmitters and hormones that can activate adenylate cyclase via G proteins; substances that inhibit phosphodiesterase, which is an enzyme that degrades cAMP, and other substances can be used to increase the intracellular cAMP concentration. It is also possible to use dibutyryl cAMP, 8-bromo-cAMP, and the like, which are a structural analogue of cAMP having the same effect as cAMP in cells.

Although not particularly limited in the present invention, examples of the cAMP inducer include forskolin (CAS No.: 66575-29-9), and forskolin derivatives (e.g., JP 2002-348243) and the following compounds. Preferably, forskolin can be used.

### Forskolin (CAS No.: 66428-89-5)

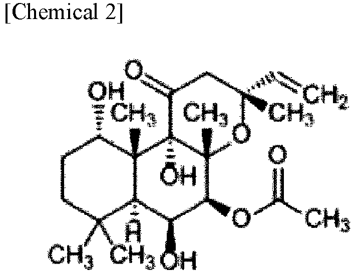

Isoproterenol (CAS No.: 7683-59-2)
NKH477 (CAS No.: 138605-00-2)
PACAP1-27 (CAS No.: 127317-03-7)
PACAP1-38 (CAS No.: 137061-48-4)

The concentration of the cAMP inducer varies depending on the kind of the inducer, the somatic cell used, and the like, but is not particularly limited and may be appropriately determined, and may be used in a range of, for example, 0.1 µmol/L to 100 µmol/L, preferably 0.5 µmol/L to 30 µmol/L.

### 2.4 BMP7

BMP (Bone Morphogenetic Protein: osteogenic proteins) 7 is one of the BMP families. Human BMP7 consists of 117 amino acid residues and activates the BMP signaling pathway by binding to cell surface receptors as a homodimer. Six types from BMP2 to BMP7 are classified as TGFb superfamilies, and are respectively encoded by distinct genes. In the present invention, either of a BMP7 derived from a human or a BMP7 derived from an animal may be used, and a recombinant thereof may be used. Among them, it is preferable to use a BMP7 derived from a human.

The concentration of the BMP7 varies depending on the kind of the BMP7, the somatic cell used, and the like, but is not particularly limited and may be appropriately determined, and may be used in a range of, for example, 0.1 ng/mL to 200 ng/mL, preferably 1 ng/mL to 50 ng/mL.

### 2.5 Preferred culture conditions

Although not particularly limited in the present invention, in the induction step, it is more preferable to culture a somatic cell in the absence of an ALK2/3 inhibitor (an agent that inhibits both ALK2 and 3) and an ALK6 inhibitor, to culture a somatic cell in the absence of an ALK5 inhibitor, or to culture a somatic cell in the absence of an ALK2/3 inhibitor, an ALK6 inhibitor, and an ALK5 inhibitor.

The absence of an ALK2/3 inhibitor, an ALK6 inhibitor, or an ALK5 inhibitor means that they are substantially absent, and it is intended to include not only the case where none of them are present at all, but also the case where they are present in such an amount that these effects are not exhibited.

ALK2 is a receptor serine/threonine kinase of ALK family members, located upstream of signaling pathways involving SMAD proteins, particularly SMAD1/5/8. Endoglin reduces the motility of prostatic carcinoma cells through activation of ALK2-Smad1 pathway. ALK2 gene is a major gene involved in fibrodysplasia ossificans progressiva (FOP), an unusual autosomal dominant congenital disorder characterized by progressive heterotopic bone formation in muscle tissue.

ALK3 is a member of the transmembrane serine-threonine kinase family. ALK3 gene acts as a minor susceptibility gene in PTEN (phosphatase and tensin homologue deleted on chromosome 10) mutationnegative Cowden's disease. ALK3 trafficking plays key roles in the pathogenesis of FOPs and is also involved in human T-cell differentiation.

Specific ALK2/3 inhibitors can include, for example, LDN193189 (CAS No.: 1062368-24-4), dorsomorphine (CAS No.: 866405-64-3), K02288 (CAS No.: 1431985-92-0), LDN212854 (CAS No.: 1432597-26-6), LDN193189 hydrochloride (CAS No.: 1062368-62-0), ML347 (CAS No.: 1062368-49-3), and LDN214117 (CAS No.: 1627503-67-6).

ALK6 is known also as BMPR1B, and is a transmembrane serine/threonine kinase member in the bone morphogenetic protein (BMP) receptor members. The ALK6 is considerably similar to activin receptors ACVR1 and ACVR2, and involved mainly in osteogenesis in a cartilage and embryogenesis.

Specific ALK6 inhibitors can include, for example, dorsomorphine (CAS No.: 866405-64-3), and K02288 (CAS No.: 1431985-92-0).

ALK5 is a TGFβ receptor subfamily member also referred to as TGFβR1 (transforming growth factor β receptor 1). The ALK5 is a serine/threonine kinase that forms a heterodimeric complex with a type II TGFβ receptor in response to binding with TGFβ, and transmits TGFβ signals from a cell surface to a cytoplasm.

Specific ALK5 inhibitors can include, for example, SB431542 (CAS No.: 301836-41-9), A83-01 (CAS No.: 909910-43-6), Repsox (CAS No.: 446859-33-2), D4476 (CAS No.: 301836-43-1), LY364947 (CAS No.: 396129-53-6), SB525334 (CAS No.: 356559-20-1), and SD208 (CAS No.: 627536-09-8).

### 2.6 Somatic Cell Culture

Culturing of somatic cells can be carried out by a conventional method using a serum-free differentiation induction medium in the presence of a selective PPARγ agonist and a cAMP inducer described above, selecting a medium, a temperature, or other conditions depending on the kind of somatic cells to be used.

Brown adipocytes (ciBAs) can be produced from somatic cells in one step by culturing the somatic cells in a serum-free differentiation induction medium containing a selective PPARγ agonist and a cAMP inducer described above.

Also, selection of somatic cells easy to culture as the somatic cells to be used allows conversion into brown adipocytes (ciBAs) of the somatic cells of which the number has been previously increased to reach a substantially confluent state.

The basal differentiation induction medium or medium for subculturing somatic cells in practicing the present invention may be selected from known or commercially available media. For example, MEM (Eagle's Minimum Essential Medium), GMEM (Glasgow's Minimum Essential Medium), DMEM (Dulbecco's Modified Eagle's Medium), Ham's F-12, DMEM/F12, RPMI1640, IMDM (Iscov's Modified Dulbecco's Medium), all of which is a general basal medium, or a medium modified therewith can be used by adding appropriate components (serum, proteins, amino acids, saccharides, vitamins, fatty acids, antibiotics, and the like).

As a culture condition, a general condition of cell culture may be appropriately selected. Examples include 37°C and 5% CO₂. It is preferred to change the medium at appropriate intervals during culture (preferably once per 1 day to 5 days, more preferably once per 2 days to 3 days). When fibroblasts are used as a stating material, brown adipocytes begin to appear in about 3 days to 7 days at 37°C, 5% CO₂, and continue to increase for up to about 21 days to 28 days.

For culturing somatic cells, a cell culture container such as a plate, a dish, a flask for cell culture, a bag for cell culture, or the like can be used. Note that, as the bag for cell culture, one having gas permeability is suitable. Large culture vessels may be used when large amounts of cells are required. The culture can be carried out in either an open system or a closed system.

Note that dexamethasone (glucocorticoid receptor agonist), insulin, 3-isobutyl-1-methylxanthine (phosphodiesterase inhibitor), triiodothyronine (thyroid hormone receptor agonist, also called T3), L-ascorbic acid 2-phosphate (ascorbic acid derivative), etc. are known to be effective agents for inducing differentiation into adipocytes. As an effective substance for inducing differentiation into brown adipocytes, for example, those commercially available as differentiation inducers can also be used. It is preferable to culture somatic cells in the presence of one or more kinds, preferably three or more kinds, more preferably four or more kinds, and even more preferably five kinds of substances selected from the above-described substances. Preferably, dexamethasone, insulin, 3-isobutyl-1-methylxanthine, T3, and L-ascorbic acid 2-phosphate may be used.

In case dexamethasone is used, the concentration of dexamethasone is not particularly limited, but it can be preferably used in a range of 0.01 µmol/L to 30 µmol/L, preferably 0.1 µmol/L to 10 µmol/L.

When insulin is used, the concentration of insulin is not limited, but can range from 1 ng/ml to 100 µg/ml, preferably 100 ng/ml to 20 µg/ml.

When 3-isobutyl-1-methylxanthine is used, the concentration of 3-isobutyl-1-methylxanthine is not particularly limited, Preferably, it can be used in the range of 1 µmol/L to 1 mmol/L, preferably 10 µmol/L to 0.5 mmol/L.

When T3 is used, the concentration of T3 is not limited, but can preferably be in the range of 1 nmol/L to 10 µmol/L, preferably 10 nmol/L to 1 µmol/L.

When L-ascorbic acid 2-phosphate is used, the concentration of L-ascorbic acid 2-phosphate is not particularly limited, but preferably 1 µg/mL to 1 mg/mL, preferably 10 µg/mL ~to 250 µg/mL can be used.

### 2.7 Inducing differentiation composition (culture medium)

Examples of the medium used for the production of ciBAs from somatic cells can include a medium in which a compound with a browning-promoting effect (e.g., TRPV1 agonist, TRPV4 antagonist, etc.), a selective PPARγ agonist and a cAMP inducer are contained as active ingredients in a basal medium prepared by mixing components required for cell culture. The active ingredients described above may be contained in a concentration effective for inducing ciBAs, and the concentration may be appropriately determined by one skilled in the art. The basal medium may be selected from known or commercially available media. For example, MEM (Minimum Essential Eagle's Medium), GMEM (Glasgow's Minimal Essential Medium), DMEM (Dulbecco's Modified Eagle's Medium), Ham's F-12, DMEM/F12, RPMI1640, and IMDM (Iscov's Modified Dulbecco's Medium), which are common media, can be used as a basal medium.

TRPV1 agonists can include, for example, capsaicin, gingerol, sanshool, allicin, piperine, camphor, nitric oxide, vanillotoxin, resiniferatoxin, and nonivamide. Among these, capsaicin is preferred. TRPV4 antagonists can include GSK2193874, RN1734, and ruthenium red.

The medium may further be supplemented with known medium components described above herein, e.g., sera, proteins (albumin, transferrin, growth factor, etc.), amino acids, saccharides, vitamins, fatty acids, antibiotics, and the like.

The medium may further be supplemented with substances effective in inducing differentiation into adipocytes such as dexamethasone, insulin, and 3-isobutyl-1-methylxanthine, T3, L-ascorbic acid 2-phosphate, as described herein above.

### EXAMPLES

Hereinafter, the present invention will be specifically illustrated by way of Examples, Comparative Examples, and Test Examples, but the present invention is not limited to the scope of the Examples and the like.

### [Example] Direct differentiation induction into ciBA

### (1) Cell culture

The human fibroblasts used as material were purchased from DS Pharma Biomedical, Co., Ltd. The fibroblasts are derived from 38-year-old human skin.

The above human fibroblasts were seeded in 35 mm dishes coated with gelatin (Cat#: 190-15805, manufactured by FUJIFILM Wako Pure Chemical Co.) at 5×10⁴, and cultured in DMEM medium (Cat#: 11965092; manufactured by Gibco) supplemented with 10% fetal bovine serum (FBS), 100 U/mL penicillin, 100 µg/mL streptomycin until 80% to 90% confluency at 37°C, 5% CO₂.

Then, after the human fibroblasts reached 80 to 90% confluency, the medium in the dish was replaced with the serum-free brown adipocyte-inducing medium "SFBAM" described below, and the human fibroblasts were cultured in SFBAM for 3 weeks. To induce ciBA, SFBAM was further supplemented with rosiglitazone (selective PPARγ agonist; 184-02651, manufactured by FUJIFILM Wako Pure Chemical Corporation) at a final concentration of 1 µmol/L, forskolin (cAMP inducer; 063-02193) at a final concentration of 7.5 µmol/L and BMP7 (bone morphogenetic protein; 026-19171, manufactured by FUJIFILM Wako Pure Chemical Corporation) at a final concentration of 20 ng/mL, and human fibroblasts were cultured for 3 weeks. Separately, capsaicin (TRPV1 agonist; 030-11353, manufactured by FUJIFILM Wako Pure Chemical Corporation) and/or nonivamide (TRPV1 agonist; sc-202735, Santa Cruz Biotechnology (manufactured by Santa Cruz Biotechnology, CA, USA), capsazepine (TRPV1 antagonist; 037-23171, manufactured by FUJIFILM Wako Pure Chemical Corporation), AMG9810 (TRPV1 antagonist; 015-25071, FUJIFILM Wako Pure Chemical Corporation), GSK1016790A (TRPV4 agonist; G0798, manufactured by Sigma-Aldrich) were added and human fibroblasts were cultured for 3 weeks. Medium was changed every 3 days.

### < SFBAM >

SFBAM is a serum-free brown adipocyte-inducing medium prepared by adding each ingredient in Table 1 to DMEM medium (Cat#: 11965092; manufactured by Gibco).

**[Table 1]**

| Ingredients | Final cone. |
|---|---|
| Triiodothyronine (T6397, by Merck) | 0.1 µmol/L |
| Dexamethasone (041-18863, by FUJIFILM Wako Pure Chemical Co.) | 0.5 µmol/L |
| IBMX (3-isobutyl-1-methylxanthine) (095-03413, by the same company above) | 30 µmol/L |
| Insulin (093-06351, by the same company above) | 6.6 µg/mL |
| L-Ascorbic acid 2-phosphate (323-44822, by the same company above) | 100 µg/mL |
| Linoleic acid-Oleic acid-Albumin (L9655, by Merck) | 1 mg/mL |
| Penicillin-Streptomycin (15070063, by Gibco) | 100 U/mL |

### (2) Evaluation experiment of browning tendency

### (2-1) RNA isolation and QRT-PCR

To quantify gene expression, total RNA was extracted from fibroblasts treated with each compound using the FastGene (registered trademark) RNA basic kit (manufactured by Nippon Genetics). As a control, fibroblasts were cultured in parallel using SFBAM and total RNA was extracted from compound-induced brown adipocytes and control cells. After reverse transcription by ReverTraAce (registered trademark) PCR RT Master Mix with gDNA Remover (manufactured by Toyobo), Real-time PCR analysis was performed using Power SYBR Green PCR Master Mix (manufactured by Thermo Fisher Scientific). Three reactions were performed per level under the following conditions.
- After 10 minutes at 95°C,
- The cycle time is 15 seconds at 95°C, followed by 60 seconds at 60°C, for 40 cycles.

All results were standardized by the amount of Tbp mRNA. The primers used in QRT-PCR are listed in Table 2 below.

**[Table 2]**

| Genes | Sense primer | Antisense primer |
|---|---|---|
| *Tbp* | ACTACGGGGTTATCACCTGTGAG (SEQ ID NO: 1) | GTGCAGGAGTAGGCCACATTAC (SEQ ID NO: 2) |
| *Ucp1* | TCTACGACACGGTCCAGGAG (SEQ ID NO: 3) | GAATACTGCCACTCCTCCAGTC (SEQ ID NO: 4) |
| *Fabp4* | GCCAGGAATTTGACGAAGTCA (SEQ ID NO: 5) | CCCATTTCTGCACATGTACCAG (SEQ ID NO: 6) |
| *Cidea* | AAGGCCACCATGTATGAGATGTAC (SEQ ID NO: 7) | ACAGGAACCGCAGCAGACTC (SEQ ID NO: 8) |

### (2-2) Immunostaining

Immunocytochemistry was performed as previously reported (Dai P, et al. J Clin Biochem Nutr. 2015; 56(3): 166-70). First, cells were fixed with 4% paraformaldehyde (manufactured by Nacalai Tesque). After washed with phosphate-buffered saline (PBS) three times, the cells were incubated with PBS containing 0.1% Triton X-100 for 10 minutes. The cells were then blocked with PBS containing 3% skim milk for 1 hour at room temperature. UCP-1 antibodies (ab10983, manufactured by Abeam, Cambridge, UK) were diluted 1/500 with the blocking solutions. The cells were incubated with the antibodies overnight at 4°C. After washed with PBS three times, the cells were incubated with Alexa Fluor 488 Donkey anti-Rabbit IgG (A-21206, manufactured by Thermo Fisher Scientific) for 1 hour at room temperature. Cell nuclei were stained with DAPI solution (manufactured by Dojindo Laboratories). All images were acquired using a fluorescence microscopy (BZ-X710-All-in-One Fluorescence Microscope, manufactured by Keyence). Fat droplets were stained using Lipi-Red (manufactured by Dojindo Laboratories).

### (2-3) Quantification of UCP1 protein

To quantify UCP1 protein, cytoplasmic proteins were extracted from fibroblasts treated with each compound using RIPA buffer (manufactured by FUJIFILM Wako Pure Chemical Corporation) with inhibitors of protease and phosphotase (manufactured by FUJIFILM Wako Pure Chemical Corporation). As a control, fibroblasts were cultured in parallel with SFBAM and cytoplasmic proteins were extracted from compound-induced brown adipocytes and control cells. The extracted proteins were electrophoresed by SDS-PAGE technique and transferred to a PVDF membrane (manufactured by Thermo Fisher Scientific). The PVDF membrane was blocked with 3% skim milk and incubated with anti-UCP1 antibodies (MAB6158, manufactured by R&D Systems, MN, USA) and anti-β-actin antibodies (A5316, manufactured by Sigma-Aldrich). Subsequently, UCP1 and β-actin each were detected using HRP-conjugated secondary antibodies (HRP-conjugated secondary antibodies Santa Cruz Biotechnology) and a chemiluminescent reagent, Immobilon Western Chemiluminescent HRP Substrate (manufactured by Merck Millipore, Darmstadt, Germany). The intensity of each detected band was quantified using ImageJ software (manufactured by National Institutes of Health).

### (2-4) Measurement method of ciBA induction efficiency

The number of Lipi-Red positive cells (adipose-like cell count), UCP1 positive cells, and Dapi positive cells (total cell count) were measured from at least three different immunostained images. From the results, the rate of adipose-like and UCP1-positive cells in the total cell count was calculated.

### (2-5) Quantification of relative mitochondrial number by QPCR

Mitochondrial DNA (mtDNA) and cellular nuclear DNA (nuDNA) were simultaneously extracted from fibroblasts treated with each compound using NucleoSpin (registered trademark) Tissue (manufactured by Takara Bio Inc). As a control, fibroblasts were cultured in parallel using SFBAM and these DNAs were extracted from compound-induced brown adipocytes and control cells. Real-time PCR analysis was performed using Power SYBR Green PCR Master Mix (manufactured by Thermo Fisher Scientific) to quantify mitochondrial DNA and cell nuclear DNA. Three reactions were performed per level under the following conditions.
- After 10 minutes at 95°C,
- The cycle time is 15 seconds at 95°C, followed by 60 seconds at 60°C, for 40 cycles.
The amount of mitochondrial DNA obtained was standardized with the corresponding amount of cell nuclear DNA. The primers used in QPCR are listed in Table 3 below.

**[Table 3]**

| Genes | Sense primer | Antisense primer |
|---|---|---|
| mtDNA | ACACCCTCCTAGCCTTACTAC (SEQ ID NO: 9) | GATATAGGGTCGAAGCCGC (SEQ ID NO: 10) |
| NuDNA | AGGGTATCTGGGCTCTGG (SEQ ID NO: 11) | GGCTGAAAAGCTCCCGATTAT (SEQ ID NO: 12) |

### (2-6) Measurement of oxygen consumption rate of brown adipocytes

To measure the oxygen consumption rate (OCR) by mitochondria, human fibroblasts were directly transformed in a serum-free medium SFBAM on 96-well plates for 3 weeks by a combination of Example 1 compounds or Example 4 compounds. As a control, human fibroblasts were cultured in a serum-free medium SFBAM in parallel. After 1 hour incubation in a non-CO₂ incubator at 37°C, the oxygen consumption rate of each well containing compound-induced brown adipocytes was analyzed using the XF96 Extracellular Flux Analyzer (manufactured by Seahorse Bioscience Inc., Billerica, MA) according to the instruction manual. During the analysis, the mitochondrial electron transfer system inhibitors oligomycin, FCCP (carbonyl cyanide 4-(trifluoromethoxy) phenylhydrazone), and antimycin A/rotenone were added to each well via an injection device at final concentrations of 2 µmol/L, 0.3 µmol/L, and 0.5 µmol/L, respectively.

### (3) Use of different human fibroblasts

Two different types of human fibroblasts shown in Table 4 below were used for cell culture as described above, and the expression levels of several marker genes (Ucp1 and Fabp4) specific for adipocytes and brown adipose tissue with respect to capsaicin were quantified by QRT-PCR.

**[Table 4]**

| | Lot # | Passage No. | BMI | Age | Gender | Depot |
|---|---|---|---|---|---|---|
| HDF35 | DFM102715B | 3 | 33.9 | 35 | Woman | Abdomen |
| HDF54 | DDFM052010B | 3 | 21.3 | 54 | Woman | Abdomen |

### (5) Results

The experimental results are shown in the drawings. Throughout the drawings, data are shown as mean ± SD (n=3), "**", "**" and "*" indicate significant differences at p < 0.001, p < 0.01 and p < 0.05, respectively, by Student's t-test, and "N.S." indicates no significant differences.

As is evident from Figures 1 to 6, 11, and 12, the browning tendency of ciBAs was enhanced by the addition of the TRPV1 agonist capsaicin. Similarly, Figures 7 to 9 clearly show that the addition of the TRPV1 agonist nonivamide also increased the browning tendency of ciBAs, while the addition of the TRPV1 antagonists capsazepine or AMG9810 suppressed the browning tendency of ciBAs.

On the other hand, the browning tendency of ciBAs was suppressed by the addition of GSK1016790A, a TRPV4 agonist, as evident from Figure 10.

Therefore, if candidate compounds are screened using ciBAs directly induced from somatic cells (fibroblasts) by low-molecular-weight compounds, it is possible to find compounds that target cell membrane receptors and that have browning-promoting or browning-inhibiting effects on brown adipocytes. In addition, candidate compounds that can act on brown adipocytes via the receptors such as TRPV1 or TRPV4, for example, can be found.

## Claims

1. A screening method of searching for a compound targeting a cell membrane receptor and having a browning-promoting or browning-inhibiting effect on brown adipocytes, wherein the method comprises a step of measuring the browning index of low-molecular-weight compound-induced brown adipocytes with and without a candidate compound applied to the brown adipocytes.

2. The screening method according to claim 1, wherein the compound having a browning-promoting or browning-inhibiting effect is a candidate compound that can act on brown adipocytes via TRPV1 or TRPV4.

3. The screening method according to claim 1 or 2, wherein the low-molecular-weight compound-induced brown adipocytes are induced to differentiate directly from a somatic cell by comprising a step of culturing the somatic cell in a serum-free differentiation induction medium in which a selective PPARγ agonist and a cAMP inducer are present.

4. The screening method according to claim 3, wherein the step comprises a step of culturing a somatic cell in the presence of BMP7.

5. The screening method according to claim 3 or 4, wherein the selective PPARγ agonist comprises rosiglitazone or the cAMP inducer comprises forskolin.

6. The screening method according to any one of claims 3 to 5, wherein the somatic cell is a fibroblast.

7. The screening method according to any one of claims 1 to 6, wherein the browning index is one or more selected from the group consisting of Ucp1 gene expression, Fabp4 gene expression, Cidea gene expression, induction efficiency of low-molecular-weight compound-induced brown adipocytes, amount of mitochondrial biosynthesis, oxygen consumption, extracellular acidification rate, amount of glycerol secretion, and amount of triglyceride accumulation.

8. A method of producing low-molecular-weight compound-induced brown adipocytes by inducing differentiation directly from a somatic cell using a compound targeting a cell membrane receptor and having a browning-promoting effect on brown adipocytes, wherein the method comprises a step of culturing a somatic cell in a serum-free differentiation induction medium in which the compound, a selective PPARγ agonist, and a cAMP inducer are present.

9. The method of producing brown adipocytes according to claim 8, wherein the compound having a browning-promoting effect comprises a TRPV1 agonist or a TRPV4 antagonist.

10. The method of producing brown adipocytes according to claim 8 or 9, wherein the step comprises a step of culturing a somatic cell in the presence of BMP7.

11. The method of producing brown adipocytes according to claim 9 or 10, wherein the selective PPARγ agonist comprises rosiglitazone, the cAMP inducer comprises forskolin, or the TRPV1 agonist comprises capsaicin.

12. The method of producing brown adipocytes according to any one of claims 8 to 11, wherein the somatic cell is fibroblast.

13. An inducing differentiation composition for producing low-molecular-weight compound-induced brown adipocytes by inducing differentiation directly from a somatic cell using a compound targeting a cell membrane receptor and having a browning-promoting effect on brown adipocytes, wherein the composition comprises the compound, a selective PPARγ agonist, and a cAMP inducer, and is serum-free.

14. The inducing differentiation composition according to claim 13, wherein the compound having a browning-promoting effect comprises a TRPV1 agonist or a TRPV4 antagonist.

15. The inducing differentiation composition according to claim 13 or 14, further comprising BMP7.

16. The inducing differentiation composition according to claim 14 or 15, wherein the selective PPARγ agonist comprises rosiglitazone, the cAMP inducer comprises forskolin, or the TRPV1 agonist comprises capsaicin.

17. The inducing differentiation composition according to any one of claims 13 to 16, wherein the somatic cell is fibroblast.
